# EUROPEAN PATENT APPLICATION

(11) **EP 2 491 927 A1**
(43) Date of publication of application: **29.08.2012**
(21) Application number: 10824499.7
(22) Date of filing: 29.09.2010
(51) Int. Cl.: A61K 31/365

(54) **USE OF A SPIROLIDE, ANALOGUES AND DERIVATIVES FOR TREATING AND/OR PREVENTING PATHOLOGICAL CONDITIONS LINKED TO THE TAU AND BETA-AMYLOID PROTEINS**

(30) Priority: 19.10.2009 ES 200930866
(71) Applicant: Universidade De Santiago De Compostela, 15782 Santiago de Compostela (ES)
(72) Inventor: BOTANA LOPEZ, Luis Miguel, E-15782 Santiago deCompostela (A Coruña) (ES); ALONSO LOPEZ, Eva, E-15782 Santiago de Compostela (A Coruña) (ES); VALE GONZALEZ, Carmen, E-15782 Santiago de Compostela (A Coruña) (ES)
(74) Representative: Pons Ariño, Angel
(86) International application number: PCT/ES2010/070633
(87) International publication number: WO 2011/048245

(57) **Abstract**

The present invention is in the field of biomedicine. Specifically, it relates to the use of a spirolide compound with the chemical structure: 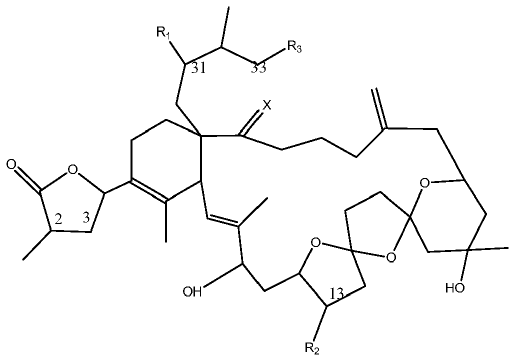 for preparing a drug for the prevention and/or treatment of a pathology related to the increase in β-amyloid protein and/or hyperphosphorylation of tau protein compared to control, where said spirolide compound is administered in the amount necessary to reach a concentration in the serum of equal to or less than 50 nM. The amount administered is preferably the amount necessary to reach a concentration in the serum of between 0.5 nM and 50 nM.

## Description

The present invention is in the field of biomedicine. Specifically, it relates to the use of a spirolide compound with the chemical structure: 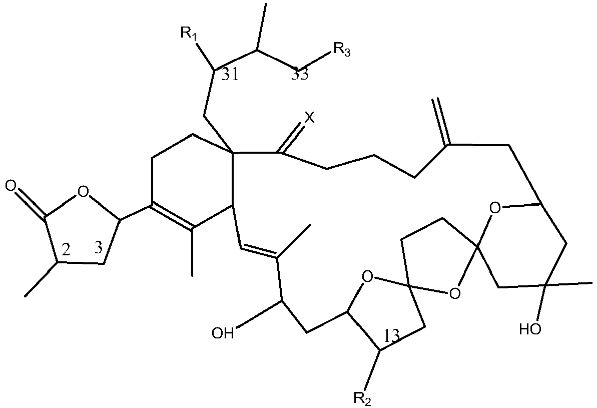 for preparing a drug for the prevention and/or treatment of a pathology related to the increase in β-amyloid protein and/or hyperphosphorylation of the tau protein compared to control, where the spirolide compound is administered in the amount necessary to reach a concentration in the serum of equal to or less than 50 nM. The amount administered is preferably the amount necessary to reach a concentration in the serum of between 0.5 nM and 50 nM.

### STATE OF PRIOR ART

Alzheimer's disease is a progressive neurodegenerative disease, of unknown origin, and for which no preventative or curative treatment can currently be offered. This disease affects between 5% and 7% of people over sixty-five years of age and is currently the most common cause of invalidity and dependence in elderly people. It is estimated that 8 million Europeans are affected by Alzheimer's disease and, taking into account the aging of the population, it is predicted that the number of sufferers will double by 2020 and triple by 2050.

This disease is characterised by a progressive loss of memory and other metal capacities as the neurones degenerate and different areas of the brain atrophy. At the neuropathological level, Alzheimer's disease is characterised by the appearance of two abnormal structures that accumulate in the brain. These structures are amyloid deposits or plaques and neurofibrillary tangles.

The amyloid deposits are insoluble fibres located intra- and extracellularly, formed by the β-amyloid (βA) peptide, more specifically by the βA40 and βA42 forms, which are generated by the sequential proteolytic cleavage of the β-amyloid precursor protein (APP) by β-secretases and γ-secretases (Shirwany et al. 2007 Neuropsychiatric Disease and Treatment; 3: 597-612). This peptide is found in the normal form in the brain in pico or nanomolar amounts. In these amounts, the peptide is in a soluble form. When an increase in β-amyloid occurs by anomalous processing of the β-amyloid precursor protein (APP), it becomes insoluble, giving rise to the formation of deposits. Various mutations in the β-amyloid precursor protein are related to Alzheimer's disease due to an increase or abnormality in the transformation of APP into β-amyloid. In patients with Alzheimer's disease, β-amyloid aggregates appear in specific cerebral regions, inducing an inflammatory response, neuronal death and progressive cognitive deterioration. This β-amyloid peptide has also been involved in neuropathological defects in individuals with Down's syndrome.

The neurofibrillary tangles by contrast are intracellular filaments formed by the polymerisation of the tau protein, which normally acts as a protein associated with neurone axon microtubules. These neurofibrillary tangles, which accumulate in the cytoplasm of degenerated neurones, were called "paired helical filaments" or PHFs. They show characteristics that are different from those of normal neurofilaments and microtubules. The main constituent of the PHFs is phosphorylated tau protein. Hyperphosphorylation of tau is due either to an increase in tau expression, because there is a higher quantity of substrate that can be phosphorylated, or by hyperphosphorylation mediated by kinases. This abnormal protein phosphorylation of tau is intimately related to abnormal aggregation of this protein. Tau hyperphosphorylation is currently involved in some 22 pathologies, including Alzheimer's disease, frontal lobe dementia (also called frontotemporal neurodegeneration), corticobasal degeneration, Pick's disease and Parkinson's disease with dementia.

Initially, studies were undertaken to try to elucidate the independent involvement of tau and β-amyloid in Alzheimer's disease. The first attempts at treatment of the disease were also directed at improving the effects of each of these proteins independently. Currently, studies carried out have shown that both proteins may be related because amyloid deposits may affect different molecular pathways that facilitate tau phosphorylation and its subsequent aggregation (Blurton-Jones et al. 2006, Current Alzheimer Research, 3(5), 435-448). Furthermore, amyloid deposits may also activate various specific kinases that increase hyperphosphorylation of the tau protein and therefore the formation of neurofibrillary tangles. Despite this relation, other studies carried out indicate that improvement in the abnormality in one of the proteins is not necessarily linked to the improvement in the other, leading in some cases to an increase in abnormality (Oddo et al., 2005. Proc Natl Acad Sci U.S.A, 102(8), 3046-51). Therefore it is necessary to carry out studies in models presenting both pathologies simultaneously.

There are currently various treatments for Alzheimer's disease that do not provide a cure for the disease but act to delay its progression. The only pharmaceutical or drug approved for the treatment of the disease when it has already advanced to a moderate or severe level, that is an advanced state, is memantine, a non-competitive antagonist of the NMDA (N-methyl-D-aspartate) receptors, which prevents the toxic effect of high glutamate concentrations in neurones. Other compounds, in this case used to prevent the development of the disease, are, for example, donepezil or rivastigmine, which act by inhibiting acetylcholinesterase, increasing the levels of the neurotransmitter acetylcholine (A. Fisher 2008, Neurotherapeutics; 5:433-442).

All this suggests that future studies of Alzheimer's disease and other neurodegenerative diseases will be directed towards the search for drugs acting on both changes and therefore leading to complete improvement of the disease.

Currently, one of the most important sources of compounds that may be useful for the production of pharmaceuticals is the marine environment. Here a multitude of biochemical resources have been found and have been demonstrated to be very useful in healthcare such as, for example, pharmaceuticals with anti-tumour activity. Among these compounds, marine phycotoxins can have extensive clinical application because of their high diversity and, therefore, multiple cellular mechanisms of action and induced responses.

Spirolides were first described in 1991 while routinely monitoring toxins in bivalves in Nova Scotia, Canada. These macrocyclic compounds were included within the group of cyclic imine toxins due to their structure. These compounds are characterised by the presence of a dimethyl group in the seventh ring. *Alexandrium ostenfeldii* (this species is also called *Goniaulax ostenfeldii, Gessnerium ostenfeldii, Triadinium ostenfeldii* or *Protogonyaulax ostenfeldii)* was identified as the microorganism responsible for the production of this toxin and is widely distributed. The toxin is characterised by having a rapid lethal toxic effect when administered by intraperitoneal injection in rodents and is much less toxic when administered orally. It should be noted that there are no known cases of toxicity in humans caused by ingestion of this compound. Investigations with the compound have shown that its target is the central nervous system, mainly the areas of the hippocampus and the brain stem, with the neurones, astrocytes and endothelial cells apparently affected. The cellular targets have been described as the muscarinic and nicotinic receptors, specifically the mAchR1, mAchR4, mAchR5, nAchRα2 and nAchRα5 subtypes.

Use of spirolides such as 13-desmethyl spirolide is known to cause toxic effects in rat and mouse neuronal cells at a concentration of 37-40 µg/kg body weight only if administered intraperitoneally and are not toxic when administered orally (Santokh et al., 2003. NeuroToxicology, 24: 593-604). Administration of spirolides has not been related to treatment of neurodegenerative diseases related to overexpression of β-amyloid protein and/or hyperphosphorylation of the tau protein.

### DESCRIPTION OF THE INVENTION

The present invention relates to the use of a spirolide compound with the chemical structure (I): 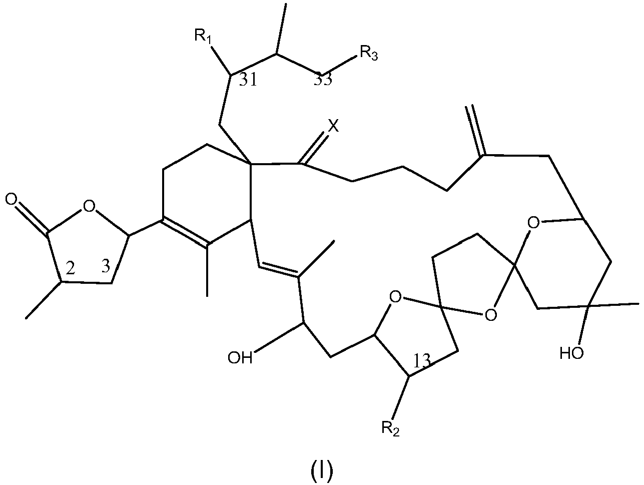 for preparing a drug for the prevention and/or treatment of a pathology related to the increase in β-amyloid protein and/or hyperphosphorylation of the tau protein compared to control, where the spirolide compound is administered in the amount necessary to reach a concentration in the serum of equal to or less than 50 nM.

In the examples of the present invention, to demonstrate the effect of spirolides on overexpression of the beta-amyloid (β-amyloid) protein and on hyperphosphorylation of the tau protein, we used *in vitro* cultures of cortical neurones obtained from triple-transgenic mice, which simultaneously overexpressed the human transgenes for presenilin (PS1_{M146V}), β-amyloid precursor protein (APP_{Swe}) and tau protein (tau_{P301}). Simultaneous overexpression of these 3 elements is related to accumulation of β-amyloid and the formation of neurofibrillary plaques and therefore these cells are useful in the study of the efficacy of compounds against diseases related to increases in the levels of the tau and β-amyloid proteins. The examples of the present invention demonstrate that treatment with spirolides causes a reduction of β-amyloid protein and tau protein phosphorylation at both the Ser202 site and the Thr212 and Ser214 sites.

The present invention demonstrates how the administration of quantities of spirolides at doses lower than those at which this compound is cytotoxic causes the same technical effect as at toxic doses, demonstrating that the reduction of the levels of tau and β-amyloid proteins can be effected by the administration of an amount of spirolides that considerably reduces the adverse effects of an excessive administration. That is, the present invention lays down the bases of the spirolide administration regime for the treatment of a pathology related to the simultaneous increase in β-amyloid protein and/or hyperphosphorylation of the tau protein. In this sense, we demonstrated that this administration does not cause a reduction in cellular viability in the neuronal model used in the study.

Specifically, the inventors have demonstrated (see the examples section) that the administration of spirolides at a concentration of 0.5 nM causes the surprising effect of reducing β-amyloid protein and hyperphosphorylation of the tau protein, this dose being 100 times lower than the toxic limit, that is, a concentration of 500 pM is able to cause beneficial effects in the treatment of diseases related to the overexpression of the β-amyloid protein and hyperphosphorylation of the tau protein.

Therefore, the present invention provides a solution to the effective treatment of neurodegenerative diseases related to the overexpression of the β-amyloid protein and/or the hyperphosphorylation of the tau protein, which are the most important elements involved in the progression of diseases such as Alzheimer's.

Spirolides are pharmacologically active macrocyclic imines that were isolated and characterised for the first time in lipophilic extracts of scallops and mussel viscera. The biogenic origin was later determined to be the dinoflagellate microorganism *Alexandrium ostenfeldii* (this species is also known as *Goniaulax ostenfeldii, Gessnerium ostenfeldii, Triadinium ostenfeldii* or *Protogonyaulax ostenfeldii).*

The spirolide of the present invention can be modified to produce derivatives that can show similar physicochemical properties. All these compounds, both analogues and derivatives, show a common chemical structure to that of spirolide (I).

Therefore, a first aspect of the invention refers to the use of a spirolide compound of chemical structure (I) for preparing a drug for the prevention and/or treatment of a pathology related to the increase in β-amyloid protein and/or hyperphosphorylation of the tau protein compared to control, where the spirolide compound is administered in the amount necessary to reach a concentration in the serum of equal to or less than 50 nM. 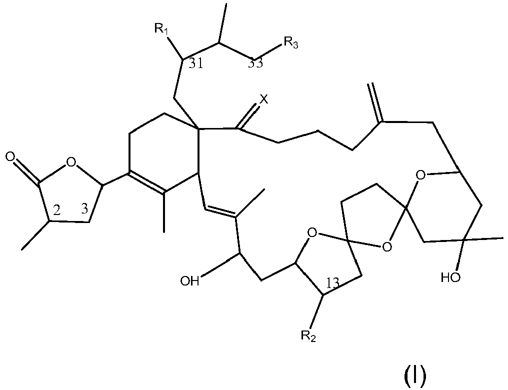 where:
- R₁ can be hydrogen or an alkyl group (C₁-C₄),
- R₂ can be hydrogen or an alkyl group (C₁-C₄),
- if C₃₃ is not linked to X and X is O, R₃ is NH₂ and
- if C₃₃ is linked to X, then R₃ is H and X is N.

As shown in the examples of the present invention, the results shown were obtained by the administration of spirolides to *in vitro* cell cultures. Therefore, the administration of the spirolide compound of the present invention to an individual must be carried out by extrapolation of the range of therapeutically effective concentrations in *in vitro* culture to a range of therapeutically effective concentrations in an individual. The therapeutically effective concentration range relates to the range in which the technical problem proposed in the present invention is resolved, that is, the prevention and/or treatment of a pathology related to the increase in β-amyloid protein and/or hyperphosphorylation of the tau protein compared to a control. Therefore, the same technical effect should be produced. This therapeutically effective concentration range can be calculated in accordance with the models of extrapolation of results *in vitro* to *in vivo* known in the state of the art. For example, an extrapolation model considers the distribution of the chemical compounds between the water, lipids and albumin of the blood serum of an individual. More specifically, an algorithm based on balanced equilibrium that requires data for albumin binding, the octanol-water partition coefficient (Kow), the albumin concentration and the lipid volume fraction, both *in vitro* and in the serum of an individual can be used for such an extrapolation (Gülden y Seibert, 2003. Toxicology, 189: 211-222).

The ways of achieving a specific range of spirolide compound concentrations in serum are known in the state of the art such as, for example but without limitation, by using controlled release pharmaceutical forms. The spirolide compound of the present invention is not restricted to any specific type of formulation. For this reason, various types of controlled or maintained release formulations may be used such as, for example, osmotic tablets, gelatinous matrix tablets, coated beads, etc.

A preferred embodiment of the present invention refers to the use of a spirolide compound of chemical structure (I), where R₁ can be hydrogen or an alkyl group (C₁-C₄); R₂ can be hydrogen or an alkyl group (C₁-C₄); and C₃₃ is linked to X, X is N and R₃ is H, that is, in this preferred embodiment, the spirolide compound refers to a compound with the chemical structure (II):

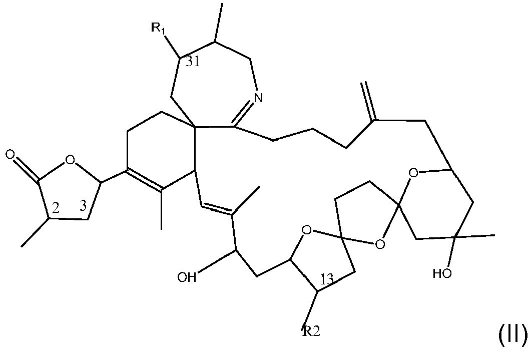

A more preferred embodiment relates to the use of a spirolide compound of chemical structure (I) where R₁ is a methyl group, R₂ is hydrogen and C₃₃ is linked to X, X is N and R₃ is H. This spirolide compound corresponds to 13-desmethyl spirolide.

Another preferred embodiment of the present invention relates to the use of a spirolide compound of chemical structure (I), where R₁ can be hydrogen or an alkyl group (C₁-C₄); R₂ can be hydrogen or an alkyl group (C₁-C₄); and C₃₃ is not linked to X, X is O and R₃ is NH₂, that is, in this preferred embodiment, the spirolide compound refers to a compound with the chemical structure (III):

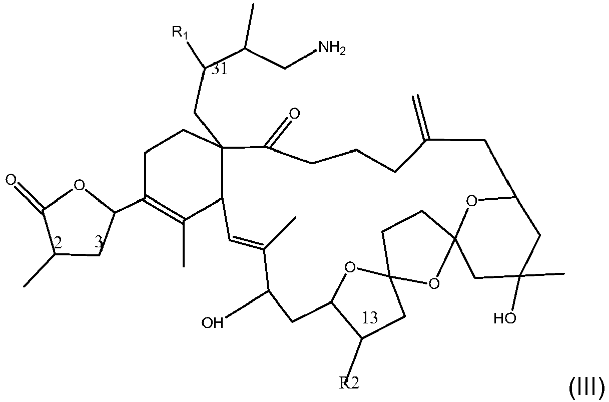

Hereinafter, to refer to any of the previously described spirolides, the expression "spirolides of the present invention" or "spirolides of the invention" can be used.

The spirolide of the present invention can be modified so that derivatives are obtained that have similar functionality. The present invention also relates to compounds analogous to the spirolide of the invention, where these compounds have a similar function to the spirolides of the invention.

Both the analogues and the derivatives have a common chemical structure to that of the spirolide of the invention (I), in which R₁ can be hydrogen or an alkyl group (C₁-C₄), R₂ can be hydrogen or an alkyl group (C₁-C₄), if C₃₃ is not linked to X and X is O, R₃ is NH₂ and if C₃₃ is linked to X then R₃ is H and X is N.

The term "analogue" as used in the present invention relates to a chemical substance similar to the spirolide of the invention in structure and/or function. For example, analogues of the spirolide of the present invention can be considered to be the spirolides A, B, C, D, E, F, G or 13-desmethyl C.

The present invention considers the term "derivative" to mean a compound that is produced starting from the spirolide of the invention by means of modifications made to it and which has a similar functionality. Such modifications can be made, for example but without limitation, by chemical, physical, microbiological or pharmacological methods.

In the present invention, the spirolide compound at various final concentrations was administered to cultured neuronal cells from 3xTg-AD mice for Alzheimer's disease with simultaneous overexpression of PS1 M146V, APP Swe and tau P301L. At these concentrations, the spirolide exercises the function of reducing the levels of the β-amyloid proteins and reducing the hyperphosphorylation of the tau protein. A concentration of 50 nM of spirolide is equivalent to 0.35 µg/ml. Assuming the density of a human body to be, for example, 950 kg/m³ (0.95 g/mL), in order to maintain a concentration of 50 nM of spirolide in a kg of "body", a total of 37 µg will be required. That is, the administration of 37 µg of spirolide per kg of body weight.

This approximate calculation of the extrapolation of the amount administered to cultured cells *in vitro* is carried out to justify the selection of the maximum concentrations administered to an individual, approximately, but is not intended to represent the only way of calculating the amount to be administered to achieve a serum concentration of equal to or less than 50 nM.

Intraperitoneal administration of an amount higher than 37 µg per kg body weight to a mouse reduces the levels of these proteins but results in toxic effects in the animal (Santokh et al., 2003. NeuroToxicology, 24: 593-604), although the *in vivo* oral toxicity of spirolides is considerably less and has been estimated to be 1 mg per kg body weight (Richard et al., 2000. Harmful Algal Blooms: 383-386). Thus, the administration of a spirolide at a dose below the lowest toxic dose according to the state of the art (with intraperitoneal administration, that is, around 37 µg per kg body weight [50 nM]), also causes the reduction of β-amyloid and reduction of hyperphosphorylation of the tau protein but without causing cytotoxic effects. The spirolide can be administered to an animal, more preferably to a mammal, including a human.

A preferred embodiment of the present invention relates to the use of the spirolide of the invention, or of its analogues or derivatives, where the said spirolide compound is administered at an amount necessary to achieve a serum concentration of between 0.5 nM and 50 nM. The inventors have demonstrated (see the examples section) that the administration of spirolides at a concentration of 0.5 nM causes the surprising effect of reducing the β-amyloid protein and hyperphosphorylation of the tau protein, this being 100 times lower than the toxic limit, that is, a concentration of 500 pM is able to produce beneficial effects in the treatment of diseases related to the overexpression of the β-amyloid protein and hyperphosphorylation of the tau protein.

A more preferred embodiment relates to the use of the spirolide of the invention, when a daily amount is administered for at least 1 day. The daily amount can be administered in various doses, preferably in one, two or three doses. According to a more preferred embodiment, the daily amount is administered in a single dose.

The dose for obtaining a therapeutically effective amount depends on a variety of factors such as, for example, age, weight, gender and tolerance of the mammal. In the sense used in this description, the expression "therapeutically effective amount" refers to the amount of pharmaceutical composition of the invention that produces the desired effect and, in general, is determined by many factors including the properties of the pharmaceutical composition and the therapeutic effect desired.

The spirolide of the present invention can be formulated together with other compounds to form part of a pharmaceutical composition or a drug. The pharmaceutical composition comprises, apart from the spirolide of the present invention, a pharmaceutically accepted vehicle or other active ingredient, or any of the combinations of the spirolide with the other components.

This pharmaceutical composition can be formulated for administration to an animal, and more preferably a mammal, including to a human, in a variety of forms known in the state of the art. Therefore, it can be administered, without limitation, in aqueous or non-aqueous solutions, in emulsions or in suspensions. Examples of non-aqueous solutions are, for example but without limitation, propylene glycol, polyethylene glycol, vegetable oils such as olive oil, or injectable organic esters such as ethyl oleate. Examples of aqueous solutions are, for example but without limitation, water, alcohol in water solutions or saline media. Aqueous solutions may be buffered or not, and can have additional active or inactive components. Additional components may include salts to modulate the ionic force, preservatives including, but without limitation, antimicrobial agents, antioxidants, chelating agents or similar, or nutrients including glucose, dextrose, vitamins or minerals. Alternatively, the compositions may be prepared for administration in solid form. Compositions may combine various inert vehicles or excipients including, without limitation: agglutinants such as microcrystalline cellulose, tragacanth gum or gelatine; excipients such as starch or lactose; dispersing agents such as alginic acid or corn starch; lubricants such as magnesium stearate; non-stick agents such as colloidal silicon dioxide; sweeteners such as sucrose or saccharine; or flavouring agents such as mint or methyl salicylate.

Any pharmaceutical composition described above and/or its formulations can be administered to an animal, including a mammal and, therefore, to a human, by a variety of routes including, but without limitation, oral, parenteral, intraperitoneal, intravenous, intradermal, epidural, intraspinal, intrastromal, intra-articular, intrasynovial, intrathecal, intralesional, intra-arterial, intracardial, intramuscular, intranasal, intracraneal, subcutaneous, intraorbital, intracapsular, topical, by transdermal patches, via rectal, via vaginal or urethral, by administration of a suppository, percutaneous, nasal spray, surgical implant, internal surgical paint, infusion pump or via catheter. A preferred embodiment of the present invention refers to the use of the spirolide of the invention where this compound is administered orally or intraperitoneally.

Increase in β-amyloid and in phosphorylation of tau, either separately or together, is often associated with a pathological process. These processes are fundamentally related to the nervous system because their accumulation basically occurs in neurones, causing their degeneration. Therefore the pathologies are neurodegenerative diseases. The main pathology where these two features occur together is Alzheimer's disease. This disease progresses with an increase in β-amyloid deposits, along with hyperphosphorylation of the tau protein giving rise to neurofibrillary tangles, that cause progressive degeneration of neurones and therefore cognitive and motor deterioration. As demonstrated in the examples, spirolide is capable of reducing overexpression of β-amyloid and hyperphosphorylation of tau, but does not have any effects on these structures if they are not altered. This indicates that these compounds are useful in the treatment of pathologies related to the increase in β-amyloid expression or hyperphosphorylation of tau both independently and together.

In the present invention, the term "prevention and/or treatment of a pathology related to the increase in β-amyloid protein compared to a control" refers to the increase in β-amyloid protein compared to the control concentration of this protein. The control concentration value of this protein is the value of this protein in a healthy individual, that is, in an individual not showing the symptoms of a pathology related to the overproduction of β-amyloid protein, known in the state of the art. The control concentration value of β-amyloid protein can also be the basal value in the same individual suffering from the disease but when they were healthy. Said value can be an average value of the concentration levels of this protein in a group of healthy individuals. Also, "hyperphosphorylation of the tau protein compared to a control" refers to a concentration of the tau protein higher than the control value of phosphorylation of this protein. The control value of phosphorylation of the tau protein is determined in the same way as has been described for the control value of the concentration of the β-amyloid protein. The determination of the concentration of the β-amyloid protein and/or phosphorylation of the tau protein is carried out in a sample isolated from an individual, the sample being preferably a biological fluid. Preferably, the biological fluid is the cerebrospinal fluid.

In the present invention, "pathology related to the increase of β-amyloid" is considered to mean any pathology featuring either an increase in levels of β-amyloid precursor protein or an increase in anomalous processing of said protein, increasing the insoluble amount and therefore giving rise to an increase in both size and quantity of intra- and extra-cellular β-amyloid deposits. Included in these pathologies, for example but without limitation, are amyotrophic lateral sclerosis, Down's syndrome, vascular dementia, cerebral amyloid angiopathy related with prion proteins and Creutzfeldt-Jakob disease. Therefore, a preferred embodiment of the present invention refers to any use of the spirolide compound of the present invention described in the previous paragraphs where the pathology related to the increase in β-amyloid is selected from the list comprising: amyotrophic lateral sclerosis, Down's syndrome, vascular dementia, cerebral amyloid angiopathy related to prion proteins and Creutzfeldt-Jakob disease.

In the present invention, "pathology related to hyperphosphorylation of tau" is considered to be any pathology featuring an increase in expression of tau, which leads to an increase in the quantity of phosphorylated protein or a hyperphosphorylation of said protein, even without change in expression, as both situations lead to an increase in the size or number of neurofibrillary tangles caused by the anomalous aggregation of phosphorylated tau. Included in the pathologies related to tau hyperphosphorylation are, for example but without limitation, frontotemporal dementia, progressive supranuclear paralysis, dementia associated with multiple system tauopathy, corticobasal degeneration and frontotemporal lobular degeneration or Pick's disease. Therefore, a preferred embodiment of the present invention relates to any use of the spirolide compound of the present invention described in the previous paragraphs where the pathology related to hyperphosphorylation of tau is selected from the list comprising: frontotemporal dementia, progressive supranuclear paralysis, dementia associated with multiple system tauopathy, corticobasal degeneration and frontotemporal lobular degeneration or Pick's disease.

There are many other diseases in addition to Alzheimer's that progress with simultaneous changes in both proteins such as, for example but without limitation, moderate cognitive disorder or deficit, hereditary cerebral hemorrhage with amyloidosis-Dutch type, cerebral amyloid angiopathy, dementia associated with Parkinson's disease, neurodegenerative disease due to diffuse Lewy bodies, corticobasal degeneration, sub-acute sclerosing panencephalitis, dementia with argyrophilic grain disease and familial Gerstmann-Straussler-Scheinker disease. Therefore, another preferred embodiment of the present invention relates to any use of the spirolide compound of the present invention described in the previous paragraphs where the pathology related to the increase in β-amyloid and hyperphosphorylation of tau is selected from the list comprising: Alzheimer's disease, moderate cognitive disorders or deficits, hereditary cerebral hemorrhage with amyloidosis-Dutch type, cerebral amyloid angiopathy, dementia associated with Parkinson's disease, neurodegenerative disease due to diffuse Lewy bodies, corticobasal degeneration, sub-acute sclerosing panencephalitis, dementia with argyrophilic grain disease and familial Gerstmann-Straussler-Scheinker disease. According to a more preferred embodiment of the present invention, the pathology related to the increase in β-amyloid protein and the hyperphosphorylation of the tau protein is Alzheimer's disease.

In the present invention, "moderate cognitive disorder or deficit" is considered to mean the change of a person's intellectual faculties that include, but without limitation, deterioration of orientation, deterioration of short term memory, deterioration of reasoning, problems with calculation, problems with language, change in the ability to carry out complex tasks and change in programming ability that appear in the initial states of different diseases such as, for example but without limitation, Alzheimer's disease, schizophrenia or senile dementia.

During the description and the claims, the use of the word "comprise" and its variants is not intended to exclude other technical characteristics, additives, components or steps. For experts in the subject, other purposes, advantages and characteristics of the invention will follow in part from the description and in part from the practice of the invention. The following examples and figures are provided for illustration purposes and are not intended as limitations of the present invention.

### DESCRIPTION OF THE FIGURES

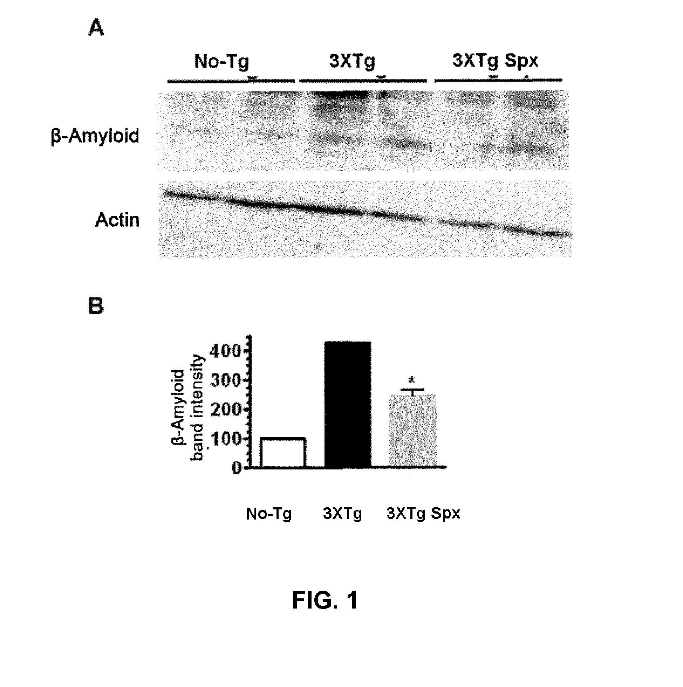

**Figure 1****. Shows the reduction in the expression levels of intracellular beta-amyloid after treatment of primary cortical cultures with spirolides.**
   (A) Western blot bands showing the expression of β-amyloid in neocortical cultures of wild animals (No-Tg), neocortical cultures obtained from 3xTgAD (3xTg) mice and levels of β-amyloid peptide in neocortical cultures of 3xTgAD mice treated with 13-desmethyl spirolide (3xTg Spx), evaluated with the 6E10 antibody. Exposure of triple-transgenic mouse cortical cultures to spirolides reduces overexpression of β-amyloid in this *in vitro* model.
   (B) Quantification of Western blot bands showing a significant reduction of overexpression of β-amyloid after treatment with 13-desmethyl spirolide (*p<0.005 compared to expression of β-amyloid in transgenic cultures, n=3, obtained in three representative experiments, each carried out in duplicate).

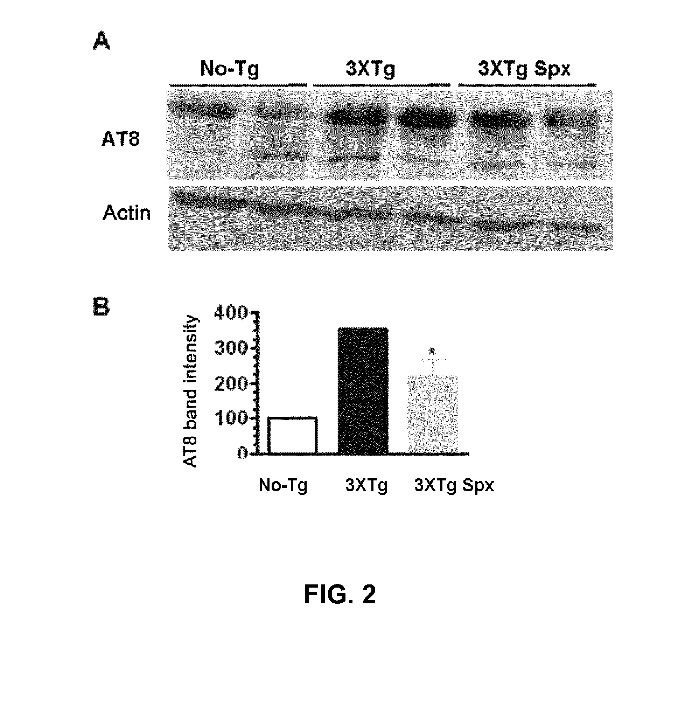

**Figure 2****. Shows a reduction in the levels of phosphorylated tau in cultures of transgenic neurones treated with spirolides.**
   (A) Western blot bands showing levels of phosphorylation of tau using the AT8 antibody (recognises phosphorylated tau on Ser202) in wild cultures (No-Tg), transgenic cultures (3xTg) and transgenic cultures treated with spirolides (3xTg Spx). Data obtained in a representative experiment.
   (B) Quantification of the expression of phosphorylated tau (marked with antibody AT8) showing a significant reduction of tau phosphorylation in transgenic cultures treated with 13-desmethyl spirolide (*p<0.05, n = 3, obtained in three representative experiments, each carried out in duplicate).

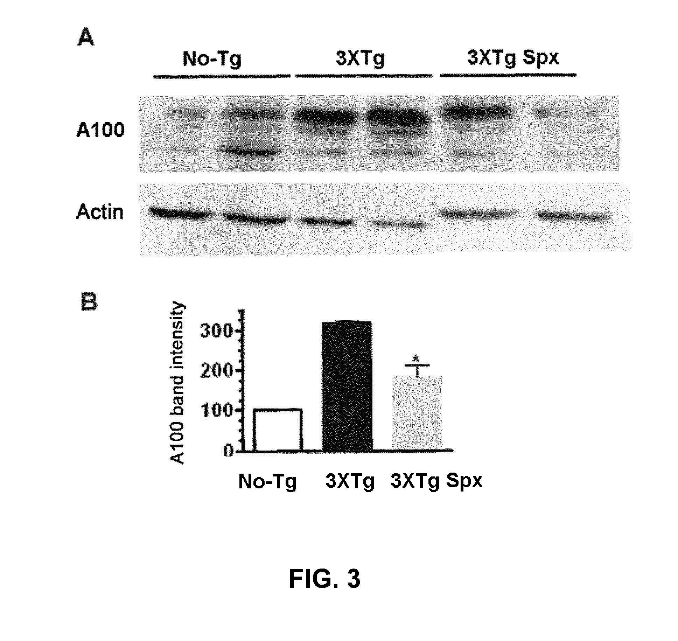

**Figure 3****. Shows inhibition of the expression of tau phosphorylated on residues Thr212 and Ser214 (marked with the AT100 antibody) in cultures of transgenic neurones treated with spirolides.**
   (A) Western blot bands showing immunoreactivity for the AT100 antibody in wild cultures (No-Tg), transgenic cultures (3xTg) and transgenic cultures treated with spirolides (3xTg Spx). Data obtained in a representative experiment.
   (B) Quantification of the expression of phosphorylated tau (marked with antibody AT100) showing a significant reduction of tau phosphorylation in transgenic cultures treated with 13-desmethyl spirolide (* p<0.005, n = 3 obtained in three representative experiments, each carried out in duplicate).

### EXAMPLES

The following specific examples provided in this patent document serve to illustrate the nature of the present invention. These examples are included only for illustrative purposes and are not to be interpreted as limitations of the invention that is claimed herein. Therefore, the examples described below illustrate the invention without limiting its field of application.

### EXAMPLE 1. DETERMINATION OF THE CELLULAR VIABILITY OF TREATMENT WITH SPIROLIDE.

In order to carry out the experiments of the present invention, either an *in vitro* cortical neuronal model with simultaneous overexpression of tau and β-amyloid, obtained from a model of Alzheimer's disease in triple-transgenic (3xTg-AD or 3xTg) mice, obtainable via the detailed procedure in the international patent application WO2003/053136 and provided by the title holders of this application, or an *in vitro* cortical neuronal model obtained from non-transgenic (No-Tg) mice. The triple-transgenic neuronal model showed overexpression of presenilin (PS1_{M146V}), β-amyloid precursor protein (APP_{Swe}) and tau protein (tau_{P301L}), which gives rise to a model of Alzheimer's disease with overexpression of β-amyloid and hyperphosphorylation of tau.

Studies in mice are representative of the effects that could be observed in humans because of the similarity of the amino acid sequences coding both for the mouse and human β-amyloid protein and tau protein. The amino acid sequence identity of the mouse β-amyloid protein (Accession No. AAA37139.1 of *Mus musculus)* and of the human (Accession No. AAC13654.1 of *Homo sapiens)* protein is 93%. Similarly the amino acid sequence identity of the mouse tau protein (Accession No. NP_034968.3 of the microtubule-associated protein tau isoform b protein of *Mus musculus)* and the human protein (Accession No. NP_058519.2 of the microtubule-associated protein tau isoform 1 protein of *Homo sapiens)* is 90%. For the percentage identity calculations, the ClustalW alignment and sequence analysis program was used, which is available on the following web page: http://www.ebi.ac.uk/Tools/clustalw2/index.html

Primary cortical cultures in the embodiment of the invention were obtained from 3xTg-AD mouse embryos of 15-17 days gestation and the wild cultures were obtained from non 3xTg control mouse embryos of the same strain. The effect of the compounds used in this invention on cellular viability was estimated by a fluorescence assay using the Alamar Blue viability indicator.

To carry out the assay, primary cortical cultures seeded on 96-well plates were used. The neuronal cells were incubated in the culture medium with the spirolide of the present invention at different concentrations dissolved in dimethyl sulfoxide and the effect on neuronal viability was determined at different treatment times *in vitro.* The maximum concentration of 13-desmethyl spirolide evaluated was 100 nM and the concentration of Alamar blue was 10%. The volume of the reaction used was 200 µl. Fluorescence was measured at wavelengths of 530 nm (excitation) and 590 nm (emission). Table 1 shows that the compounds of the present invention did not change *in vitro* cellular viability.

**Table 1: Viability of cortical neurones after treatment with different concentrations of spirolide for 72 hours in culture. The data are means ± standard error of three independent experiments and are expressed as percentage of control.**

| | **% control** |
|---|---|
| Control | 100 ± 5.3 |
| 2.5 nM spirolide | 104.3 ± 1.2 |
| 5 nM spirolide | 104 ± 1.3 |
| 10 nM spirolide | 104.3 ± 1.4 |
| 25 nM spirolide | 104.3 ± 0.7 |
| 50 nM spirolide | 103.4 ± 0.5 |
| 100 nM spirolide | 102.1 ± 1.5 |

The administration of 50 nM spirolide is equivalent to approximately 37 µg spirolide per kg of body weight. Intraperitoneal administration of an amount higher than 37 µg per kg body weight to a mouse reduced the levels of these proteins but produced toxic effects in the animal (Santokh et al., 2003. NeuroToxicology, 24: 593-604), however, the oral toxicity of spirolides is 1 mg per kg body weight. The investigators have evaluated the effect of spirolides at amounts less than 0.5 µg per kg body weight (approx. 0.67 nM), observing that at 0.017 µg per kg body weight (approx. 0.023 nM) the hyperphosphorylation of the tau protein continued to be reduced. Table 2 shows the effects of different concentrations of spirolides on the levels of phosphorylated tau, determined with the AT8 antibody (recognises tau phosphorylated on Ser202).

**Table 2. Effect of different concentrations of spirolide on the levels of tau phosphorylated on Serine 202. The cell cultures were exposed to different concentrations of spirolide from day 3 to day 10 in vitro. The data represent the mean ± se of two experiments.**

| | **% control** |
|---|---|
| Control | 100 ± 2.1 |
| 3xTg | 129.1 ± 1.6 |
| 3xTg-Spx 25 nM | 89.1 ± 2.2 |
| 3xTg-Spx 10 nM | 70.3 ± 4.7 |
| 3xTg-Spx 0.5 nM | 61.7 ± 12.4 |
| 3xTg-Spx 0.1 nM | 129.8 ± 1.7 |

### EXAMPLE 2. EFFECT OF 13-DESMETHYL SPIROLIDE ON OVEREXPRESSION OF INTRACELLULAR β-AMYLOID AND HYPERPHOSPHORYLATION OF TAU.

For the determination of the effect of the spirolide compound of the present invention on the expression of tau and beta amyloid, the Western blot techniques were used. The primary neuronal cultures were incubated with the spirolide compound of the present invention added to the culture medium for a specific time. For the concentration of 50 nM, the treatment times were between 7 and 10 days *in vitro* and for other concentrations (0.1 to 25 nM) between 3 and 10 days *in vitro.* The cells were then processed following the usual protocols for Western blot. For Western blot studies, protein expression was evaluated using 6E10 anti-β-amyloid primary antibodies at a dilution of 1:500, AT8 anti-Tau (tau phosphorylated on Ser202, dilution 1:1000) and AT100 anti-tau (tau phosphorylated on Thr 212 and Ser214, dilution 1:1000).

For Western blot assays, neuronal cultures treated with the spirolide of the present invention were washed with cold phosphate buffer and lysed in 50 mM Tris-HCl buffer (pH 7.4) containing 150 mM NaCl, 1 mM EDTA, 1% Triton X-100, 2 mM DTT, 2.5 mM PMSF, 40 mg/ml aprotinin, 4 mg/ml leupeptin, 5 mM NaF, 1 mM Na₃VO₄, 1 mg/ml pepstatin A and 1 mg/ml benzamidine. Total protein concentration was determined by Bradford's method using bovine albumin as standard. Aliquots of cellular lysates containing 20 µg total protein were loaded in loading buffer (50 mM Tris-HCl, 100 mM dithiothreitol, 2% SDS, 20% glycerol, 0.05% bromophenol blue, pH 6.8), the proteins were separated by electrophoresis and transferred to PVDF membranes. The membranes were incubated with the primary antibodies, washed and then incubated with a secondary antibody bound to HRP. Immunoreactivity was detected by chemiluminescence. The same membranes were reincubated with an anti-β-actin primary antibody to carry out data correction for the sample protein content. These assays demonstrated that treatment with 13-desmethyl spirolide reduced overexpression of β-amyloid (Fig. 1) and tau phosphorylated on the Ser202 (Fig. 2) and on the Thr212 and Ser214 (Fig 3) sites in neurones obtained from triple transgenic mice.

### EXAMPLE 3. EFFECT OF THE COMPOUNDS OF THE PRESENT INVENTION ON BASAL PHOSPHORYLATION OF TAU IN CONTROL NEURONES.

The effect of spirolide on basal phosphorylation of tau was determined by Western blot techniques. An *in vitro* cortical neuronal model obtained from non-transgenic mice was used. Neuronal culture and sample processing was carried out in the same way as described in the previous example. The data obtained were corrected for the β-actin content of the samples. The data obtained demonstrated that treatment of control neurones with a concentration of 100 nM spirolide did not affect the basal amount of total tau (determined with the Tau46 antibody that recognises different isoforms both of phosphorylated and non-phosphorylated tau, Table 3).

**Table 3: Effect of 13-desmetyl spirolide on total basal tau levels in control neurones. The data are means ± standard error of two independent experiments and are expressed as percentage of non-treated control.**

| | **% control** |
|---|---|
| Control | 100 % |
| Control + 100 nM 13-desmethyl spirolide | 139.9 ± 27.3 |

### EXAMPLE 4. EFFECT OF 13-DESMETHYL SPIROLIDE ON THE LEVELS OF EXTRACELLULAR β-AMYLOID.

The levels of extracellular β-amyloid in the culture medium of transgenic and control cortical neurones treated with the spirolide of the present invention were determined by commercial ELISA kits for the detection of β-amyloid 1-42 or β-amyloid 1-40 following the instructions recommended by the manufacturer. These kits consist of plates treated with antibodies that capture the β-amyloid peptide by its amino terminal end. The templates with known amounts of β-amyloid and the culture medium recommended for neuronal preparations with unknown amounts of β-amyloid were added to the wells and were incubated. The plates were washed to remove the unbound peptide and were incubated for 2 hours in the presence of an antibody that binds to the carboxyl terminal end of the β-amyloid peptide. Next, the plates were washed and incubated with a secondary antibody bound to peroxidase. After 25 minutes of incubation, the plates were washed and the OPD substrate was added to visualise the bound peptide. The optical density was measured by absorbance at 495 nm and a straight line was obtained with the amounts of β-amyloid added, from which the amount of β-amyloid in the culture medium recovered from neuronal preparations treated with the spirolide of the present invention was extrapolated.

All the procedures described in the present invention can be automated for HTS (high throughput screening) using culture plates to which the cells, which are cultivated for at least 6 days and subjected to treatment with the spirolide of the present invention, have been previously added. These plates may be incorporated into automatic measurement systems of the markers of interest by various measurement methods (absorbance, luminescence, fluorescence). Similarly, the effect of the spirolide of the present invention, alone or in combination with other agents in cellular, molecular or biochemical targets, of relevance for Alzheimer's disease and/or other neuropathological changes that feature changes in the phosphorylation of tau or in the expression of β-amyloid, can be investigated by HTS methods using commercial kits of interest for the evaluation of the effect of these treatments in the progress of Alzheimer's disease or diseases associated with tau and β-amyloid, after the cells have been subjected to the treatment in question.

## Claims

1. Use of a spirolide compound of chemical structure (I) 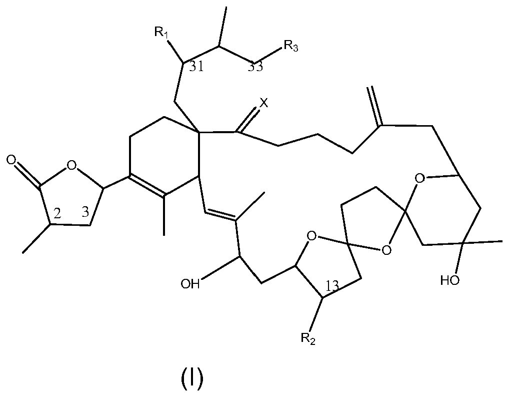 wherein:
R₁ can be hydrogen or an alkyl group (C₁-C₄),
R₂ can be hydrogen or an alkyl group (C₁-C₄),
if C₃₃ is not linked to X and X is O, R₃ is NH₂ and
if C₃₃ is linked to X, then R₃ is H and X is N,
for preparing a medicament for the prevention and/or treatment of a pathology related to the increase in β-amyloid protein and/or
hyperphosphorylation of the tau protein compared to control, where said spirolide compound is administered in the amount necessary to reach a concentration in the serum of equal to or less than 50 nM.

2. Use of a spirolide compound according to claim 1, wherein C₃₃ is linked to X, X is N and R₃ is H.

3. Use of a spirolide compound according to claim 2, wherein R₁ is a methyl group and R₂ is hydrogen.

4. Use of a spirolide compound according to claim 1, wherein C₃₃ is not linked to X, X is O and R₃ is NH₂.

5. Use of a spirolide compound according to any of claims 1 to 4, wherein said spirolide compound is administered in an amount necessary to reach a concentration in the serum of between 0.5 nM and 50 nM.

6. Use according to any of claims 1 to 5, wherein said amount is administered daily for at least 1 day.

7. Use of a spirolide compound according to claim 6, wherein the daily amount is administered in a single dose.

8. Use of a spirolide compound according to any of claims 1 to 7, wherein said compound is administered orally or intraperitoneally.

9. Use of a spirolide compound according to any of claims 1 to 8, wherein the pathology related to the increase in β-amyloid protein is selected from the list comprising: amyotrophic lateral sclerosis, Down's syndrome, vascular dementia, cerebral amyloid angiopathy related to prion proteins and Creutzfeldt-Jakob disease.

10. Use of a spirolide compound according to any of claims 1 to 9, wherein the pathology related to the hyperphosphorylation of the tau protein is selected from the list comprising: frontotemporal dementia, progressive supranuclear paralysis, dementia associated with multiple system tauopathy, corticobasal degeneration and frontotemporal lobular degeneration and Pick's disease.

11. Use of a spirolide compound according to any of claims 1 to 10, wherein the pathology related to the increase in β-amyloid protein and hyperphosphorylation of the tau protein is selected from the list comprising:
Alzheimer's disease, moderate cognitive disorders or deficits, hereditary cerebral hemorrhage with amyloidosis-Dutch type, cerebral amyloid angiopathy,
dementia associated with Parkinson's disease, neurodegenerative disease due to diffuse Lewy bodies, corticobasal degeneration, sub-acute sclerosing panencephalitis, dementia with argyrophilic grain disease and familial Gerstmann-Straussler-Scheinker disease.

12. Use of a spirolide compound according to claim 11, wherein the pathology related to the increase in β-amyloid protein and hyperphosphorylation of the tau protein is Alzheimer's disease.
